# EUROPÄISCHE PATENTSCHRIFT

(11) **EP 3 272 386 B1**
(45) Veröffentlichungstag und Bekanntmachung des Hinweises auf die Patenterteilung: **23.10.2024**
(21) Anmeldenummer: 17189242.5
(22) Anmeldetag: 22.07.2010
(51) Int. Cl.: A61M 27/00, A61M 1/00

(54) **VAKUUMSCHWAMMDRAINAGE**
VACUUM SPONGE DRAINAGE
DRAINAGE PAR ÉPONGE SOUS VIDE

(30) Priorität: 30.09.2009 DE 102009043472; 09.12.2009 DE 102009057374; 29.03.2010 DE 102010013271; 30.03.2010 DE 102010013439; 01.04.2010 DE 102010013849; 01.04.2010 DE 102010013848
(43) Veröffentlichungstag der Anmeldung: 24.01.2018
(62) Teilanmeldung aus: 10739328.2
(73) Patentinhaber: Lohmann & Rauscher GmbH & Co. KG, 56567 Neuwied (DE)
(72) Erfinder: Schorsch, Tobias Paul Heinrich, 22095 Hamburg (DE); Loske, Gunnar, 22926 Ahrensburg (DE)
(74) Vertreter: Herrmann, Daniel

(56) Entgegenhaltungen:
- WO-A1-2004/041346
- WO-A2-03/028786
- WO-A2-2006/114637
- DE-A1- 102008 061 535
- US-A- 6 123 697
- US-A1- 2007 219 497
- US-A1- 2007 282 310

## Beschreibung

Die Erfindung betrifft ein Vakuumschwammsystem zur Verwendung im menschlichen oder tierischen Körper.

WO 2004/041346 A1 beschreibt ein endoskopisches Wundpflegebehandlungssystem. Die WO 2004/041346 A1 bezieht sich insbesondere auf eine endoskopische Komponente, die die Anwendung der Unterdrucktherapie auch im Inneren eines menschlichen oder tierischen Körpers ermöglicht.

In der US 2007/0219497 ist ein System für die Unterdrucktherapie im Bereich der Knochen angegeben. In der US 6,123,697 ist ein Verfahren zum Absorbieren von Fluiden unter Anlegen eines Drucks beschrieben.

Der Erfindung liegt die Aufgabe zugrunde, eine Weiterentwicklung der WO 2004/041346 A1 bereitzustellen, die die Nachteile der bekannten Systeme beseitigt oder wenigstens reduziert.

Erfindungsgemäß wird die Aufgabe durch ein Vakuumschwammsystem mit den Merkmalen des Patentanspruchs 1 gelöst.

Der Erfindung liegt die Erkenntnis zugrunde, dass die Verwendung der Vorrichtung der WO 2004/041346 A1 in Bereichen innerhalb des menschlichen oder tierischen Körpers tiefer als ca. 10 cm (von der Körperöffnung aus gesehen) im menschlichen oder tierischen Körper zu Anwendungsproblemen führt. Ferner hat der Erfinder erkannt, dass das Anwendungsgebiet der Vorrichtung der WO 2004/041346 A1 sich insbesondere auf nur einseitig offene Bereiche innerhalb des Körpers beschränkt und bei Verwendung in zweiseitig offenen, kanalartig ausgebildeten Bereichen innerhalb des Körpers problematisch ist.

Der Erfinder, Dr. Gunnar Loske, hat bei seiner Arbeit im Marienkrankenhaus, Hamburg, Deutschland eine Weiterentwicklung der Vorrichtung der WO 2004/041346 A1 erfunden. Die Weiterentwicklung erreicht vorteilhaft, dass die erfindungsgemäße Vakuumschwammeinheit für Bereiche innerhalb des menschlichen oder tierischen Körpers geeignet ist, die insbesondere tiefer als 10 cm (von der Körperöffnung aus gesehen) im menschlichen oder tierischen Körper gelegen sind, und dass die Vakuumschwammeinheit nicht nur in einseitig offenen Bereichen innerhalb des Körpers, sondern besonders vorteilhaft in zweiseitig offenen, kanalartig ausgebildeten Hohlräumen (insbesondere in dem gesamten Magen-Darm-Trakt) Innerhalb des Körpers verwendet werden kann.

Der Erfindung liegt die Idee zugrunde, dass ein Kanal Innerhalb des Fluidsammelelements, das insbesondere eine Schwammeinheit ist, diese und weitere Vorteile erreichen kann.

Der Kanal kann dazu genutzt werden, um ein Versorgungselement In den Körper einzuführen. Die Vorrichtung der WO 2004/041346 A1 bietet den Nachteil, dass wenn sie im gesamten Magen-Darm-Trakt verwendet werden würde, eine Ernährung oder Darmleerung nur nach Entfernen des Fluidsammelelements möglich wäre. Erfindungsgemäß wird dieser Nachteil dadurch beseitigt, dass der Kanal ein Versorgungselement beispielsweise zum Ernähren nicht entfernt werden muss.

Der Kanal wird dazu genutzt, um ein Versorgungselement in dem Körper angeordnet zu belassen, In der WO 2004/041346 A1 wird das Endoskop zunächst aus dem Körper entfernt und anschließend das Fluidsammelmittel eingebracht. Eine optische Überwachung mittels des Endoskops ist also nach Positionierung und Aktivierung des Fluidsammelmittels nicht mehr bzw. nur sehr aufwendig möglich. Die Erfindung behebt diesen Nachteil, indem der Kanal Platz für beispielsweise ein Endoskop bietet, so dass eine gleichzeitige Nutzung von Fluidsammelelement und Endoskop möglich ist. Auch ist es möglich, bei oder nach Aktivierung des Fluidsammelelementes beispielsweise ein Endoskop vorzusehen/einzuführen, um den Zustand des zu versorgenden Körperbereichs zu überwachen und die Platzierung zu kontrollieren.

Das Fluidsammelelement ist besonders bevorzugt eine Schwammeinheit. Die Schwammeinheit erfüllt die Funktion eines Ab- und Zuleitens von Flüssigkeiten und Gasen in und aus dem Körper. Dies ist insbesondere die Funktion einer Druckverteilung, so dass ein an das Fluidkommunikationselement angelegter Unterdruck ein Kollabieren der Schwammeinheit und des diese umgebenden Körperbereichs veranlasst.

Durch die Einlage eine Drainage verbunden mit einem offenporigen Polyurethanschwamm lassen sich bekanntermaßen innere Wunden bei einer postoperativen Anastomoseninsuffizienz am Enddarm behandeln. Die Drainage wird hierzu unter Sog gesetzt, sie entfernt das Wundsekret, führt zur Wundverklebung und Abheilung. Durch das bislang verfügbare Einführsystem lassen sich nur körperöffnungsnahe Wunden versorgen.

Mit der Erfindung zu erzielenden Vorteile liegen in der tieferen Positionierung einer Schwammdrainage und der sich hierdurch ergebenden erweiterten Indikationsstellung der Vakuumschwammtherapie. Das Schwammsystem lässt sich mit der Erfindung auch in Speiseröhre und Magen einlegen, die mit einem Endoskop erreicht werden können. Eine Speiseröhrenperforation kann endoskopisch durch die Versorgung mittels eines gecoverten selbstexpandierenden Stents erfolgen. Dieser wird mit einem Einführsystem über einen zuvor endoskopisch eingelegten Führungsdraht über dem Defekt eingebracht und soll diesen von innen abdichten. Hierzu ist auch eine von außen eingelegte Wunddrainage notwendig, Mit der Erfindung wird über einen endoskopisch eingelegten Führungsdraht das Einführsystem positioniert und ein offenporiger Polyurethanschwamm, welcher mit einer Drainage verbunden ist, über dem Perforationdefekt der Speiseröhre eingelegt. Die Drainage wird mit einer Vakuumpumpe unter einen Unterdruck gesetzt. Hierdurch kollabiert das Speiseröhrenlumen über dem Schwamm, der Defekt wird verschlossen und Wundsekret gleichzeitig drainiert. Der Vorteil dieses Verfahrens gegenüber dem Stentsystem ist die Drainage des Wundsekretes nach innen und die Ableitung über die Drainage durch die Sogwirkung in Richtung des Darmlumens. Dieses ist insbesondere wirkungsvoll an Organen die natürlicherweise unter einem Unterdruck stehen (Organe in der Brusthöhle) oder Wunden und Wundhöhlen die mit der Brusthöhle in Kontakt stehen und sich hier durch physiologischerweise unter einem Unterdrück befinden. Durch den Vakuumsog, der über die Drainage an den offenporigen Polyurethanschwamm angelegt wird, kann sich das Wundsekret über die Drainage entleeren und wird nicht entlang des physiologischerseits bestehenden Druckgradienten und bei Atembewegungen in Richtung des Brustkorbes gezogen.

Bei der endoskopischen Untersuchung des Magendarmtraktes, insbesondere der Speiseröhre und des Magen, aber auch des Dünndarmes werden Schutzhülsen, sogenannte Overtubes benutzt. Sie werden vor der Untersuchung über das Endoskop geschoben und nach dem Einführen des Endoskops in das Darmlumen über dem liegenden Endoskop in den Darm nachgeführt. Overtubes dienen nach ihrer Platzierung der sicheren Überbrückung des Rachenraumes und der Speiseröhre bei endoskopischen Behandlungen. Insbesondere wenn das Einführen eines Endoskops im Rachenraum und der oberen Speiseröhre schwierig ist, und ein Endoskop bei der Untersuchung wiederholt ein- und ausgeführt werden muss, können Verletzungen des Rachens und der Speiseröhrenwand vermieden werden, da der Overtube als Schutzhülsenschienung für das Endoskop dient. Overtubes werden eingesetzt, um bei der endoskopischen Entfernung von Fremdkörpern Verletzungen der Darmwand zu verhindern. Bei der endoskopischen Untersuchung des Dünndarms dienen Overtubes als Schienung und Schutz bei der Überbrückung von Speiseröhre, Magen und Dünndarm sowie als technisches Hilfsmittel zur Erreichung tieferer Darmabschnitte. Die bislang verfügbaren Overtubes werden vor einer endoskopischen Untersuchung auf dem Endoskop aufgeschoben, anschließend wird über eine Körperöffnung eingespiegelt, der Overtube über dem Endoskop nachgeschoben und als Schienung platziert. Die endoskopische Untersuchung wird dann über dem liegenden Overtube vorgenommen. Flexible endoskopische Operationen werden derzeit über spezielle Instrumente ausgeführt, die über Arbeitskanäle des flexiblen Endoskops eingeführt werden. Bei endoskopischen Operationen mit starren Endoskopen, z. B. bei einer Bauchhöhlenspiegelung, wird eine starre Geräteoptik über einen Trokar in eine Körperhöhle eingebracht und weitere zusätzliche starre Instrument über gesonderte Inzisionen über Trokare eingesetzt. Neuerdings werden zusammen mit der Geräteoptik Operationsinstrumente'über einen einzelnen Zugang (Singleport) eingesetzt.

Bevorzugt ist die Drainageleitung ein Schlauch. Bevorzugt besitzt die Drainageleitung am distalen Ende seitliche Öffnungen. Bevorzugt besitzt die Drainageleitung am distalen Ende seitliche Öffnungen über die gesamte oder inkomplette Länge des Fluidsammelmittels. Bevorzugt Ist eine zusätzliche Drainageleitung in den Schwamm eingeführt. Bevorzugt ist die zusätzliche Drainageleitung in dem Fluidmittel verschieblich. Bevorzugt Ist die zusätzliche Drainageleitung in dem Fluidmittel fest befestigt. Bevorzugt ist die zusätzliche Drainageleitung an der distalen Spitze abgerundet. Bevorzugt beinhaltet auch die zusätzliche Drainageleitung ein Führungselement. Bevorzugt verjüngt sich das Fluidsammelmittel am distalen Ende konisch. Bevorzugt verjüngt sich das Fluidsammelmittel am proximalen Ende konisch. Bevorzugt sind mehrere Fluidsammelmittel auf einem Drainageschlauch befestigt. Bevorzugt ist die Schwammdrainage zur Intraluminalen und intracavitären Vakuumschwammtherapie Im gesamten Magendarmtrakt, zur Platzierung über natürliche Körperöffnungen mit gleichzeitiger Möglichkeit der zusätzlichen Ernährung bei intraluminaler Schwammplatzierung ausgestaltet.

An der Speiseröhre konnten Defekte durch Perforationen oder postoperativen Anastomoseninsuffizienzen durch die intraluminale Einlage einer Polyurethanschwammdrainage behandelt werden. Eine Schwammdrainage wurde dazu über dem Defekt In das Intestinallumen eingelegt. Wenn die Drainage dann unter Sog gesetzt wird, verschließt sich das Darmlumen, der Defekt kann verkleben und heilen. Ebenso konnte auch am Zwölffingerdarm die Wirksamkeit der intraluminalen Therapie gezeigt werden. Mit der Vakuumschwammtherapie ist eine endoskopische Behandlung dieser Verletzungen möglich. Durch das bislang verfügbare Einführsystem lassen sich nur körperöffnungsnahe Anastomoseninsuffizienzen im Enddarm versorgen.

Die mit der Erfindung erzielbaren Vorteile und neuen Behandlungsmöglichkeiten sind zahlreich. Bei der Erfindung handelt es sich um eine Schwammdrainage, welche unter endoskopischer, radiologischer oder operativer Sicht platziert wird. Die Platzierung erfolgt über ein Überschieben der Drainage über einem zuvor endoskopisch eingelegten Führungsdraht. Endoskopisch wird ein Führungsdraht intraluminal eingelegt und anschließend unter endoskopischer, radiologischer oder operativer Kontrolle die Schwammdrainage entlang des Führungsdrahtes exakt zum gewünschten Platzierungsort geschoben. Die Erfindung kann intraluminal im gesamten Magendarmtrakt eingelegt werden. Durch die Einlage eines zusätzlichen Drainageschlauches in den Schwamm, eröffnen sich neue Behandlungsmöglichkeiten. Wenn dieser zusätzliche Drainageschlauch in dem Schwamm verschieblich konstruiert wird, kann er nach der Positionierung der Schwammdrainage vorgeschoben oder auch entfernt werden. Wenn z. B. eine Behandlung einer Speiseröhrenverletzung vorgenommen wird, kann der zusätzliche Drainageschlauch in den Magen vorgeschoben werden und hierüber eine enterale Ernährung oder eine Dekompression zur Entlastung des Magens erfolgen. Diese Verfahren sind auch dann möglich, wenn der Schwamm unter Unterdruck gesetzt wird und das Speiseröhrenlumen verschließt. Durch den Unterdruck saugt sich zum einen der Schwamm im Darmlumen fest, so dass er sich nicht verschiebt, zum anderen saugt er auch die zusätzliche Drainage fest und fixiert diese im Schwammdurchtritt.

Bevorzugt besitzt das Fluidsammelmittel eine Röntgenmarkierung. Bevorzugt ist das Fluidsammelmittel röhrenförmig mit einem zentralen Lumen konstruiert. Bevorzugt umfasst das Fluidsammelmittel eine elastisch komprimierbare Struktur mit Fluidkanälen. Bevorzugt umfasst das Fluidsammelmittel einen offenporigen Polyurethanschwamm. Bevorzugt sind eine oder mehrere Drainageleitungen in dem Fluidsammelmittel befestigt. Bevorzugt sind die Drainageleitungen Schläuche. Bevorzugt besitzt die Drainageleitung am distalen Ende seitliche Drainageöffnungen. Bevorzugt ist die Drainageleitung am distalen Ende über die gesamte Länge, die sich in dem Fluidmittel befindet, mit seitlichen Drainageöffnungen versehen. Ferner bezieht sich die Erfindung auf einen Overtube für ein vorstehend erläutertes Endoskop, wobei der Overtube aus einem Kunststoffschlauch besteht.

Zur Behandlung von sekundär heilenden Wunden werden im Stand der Technik offenporige Polyurethanschwämme in Wunden eingelegt, mit einer Folie nach außen verschlossen, mit einem Drainageschlauch verbunden und mit Hilfe eines Vakuumpumpensystems unter Sog gesetzt. Die Wunde kollabiert unter dem Unterdruck, das Wundsekret wird von der Wunde kontinuierlich oder intermittierend abgesaugt. Die Wunde kann sich rasch reinigen, verkleinern und Granulationsgewebe ausbilden. Dass dieses Therapiekonzept auch an inneren Wunden effektiv ist, konnte an Wunden in Folge von Nahtrupturen nach Enddarmoperationen nachgewiesen werden. Hierzu wird eine Polyurethanschwammdrainage durch die Nahtruptur hindurch in eine dahinterliegende Wundhöhle eingebracht.

Ebenso konnte gezeigt werden, dass die Vakuumschwammtherapie auch in tieferen nur mit einem Endoskop zu erreichenden inneren Wunden z. B. als Folge von Operationen oder Verletzungen der Speiseröhre effektiv ist. Dazu wird eine Vakuumschwammdrainage unter endoskopischer Sicht entweder über einen Defekt in eine Wundhöhle eingebracht.

Durch die Erfindung wird die endoskopische Platzierung einer Vakuumschwammdrainage technisch einfach. Die neuen medizinischen Behandlungsmöglichkeiten, die sich durch die Drainage von inneren Wunden oder die Herbeiführung eines Verschlusses des Intestinallumens herbeiführen lassen, sind vielfältig.

Bei der Erfindung handelt es sich um einen offenporigen röhrenförmig konstruierten Polyurethanschwammkörper, variabler Länge. Dieser Schwamm wird mit dem Distalende eines Drainageschlauches, welcher am distalen Ende Perforationsöffnungen besitzt, fest verbunden. Der Schwammkörper wird auf einem, durch einen Overtube geführtes, Endoskop aufgesetzt, er ist auf diesem verschieblich. Bei dem Overtube handelt es sich um eine auf dem Endoskop verschiebliche schlauchartige Hülse. Der Overtube liegt proximal der röhrenförmigen Schwammdrainage. Zum einen verhindert er, dass der Schwamm auf dem Endoskop nach proximal rutschen kann. Zum anderen wird der Overtube als Pusher dazu benutzt, die Schwammdrainage auf dem Endoskop gleitend, nach distal zu führen und vom Endoskop abzuschieben und so freizusetzen. Es kann auf diese Weise eine Schwammdrainage sowohl in eine Wund- oder Körperhöhle oder z. B. intraluminal in einem Darmlumen oder Hohlorgan eingebracht werden. Sobald die Schwammdrainage freigesetzt ist, wird ein Unterdruck an diese angelegt, so dass sie sich festsaugt. Dann kann das Endoskop entfernt werden.

Vor der Schwammplatzierung ist es im selben Arbeitsschritt möglich über den Arbeitskanal eines Endoskops eine Sonde vorzuschieben. Bei einem endoskopischen Einlegen einer Schwammdrainage In der Speiseröhre, kann über diese z. B. bis in Magen oder Dünndarm eingebrachte , erfindungsgemäße Sonde, eine enterale Ernährung oder die Verabreichung von oralen Medikamenten erfolgen.

Bevorzugt bezieht sich die Erfindung auf ein Drainagesystem zum Absaugen von Körperflüssigkeiten, Wundsekreten, Gasen aus Körperhöhlen, Hohlorganen, Gewebeabszessen, Intestinallumina, mit Drainageleitungen, Fluidsammelmittel und Verbindungselementen für die Drainageleitungen. Bevorzugt umfasst das Fluidsammelmittel eine elastisch komprimierbare Struktur mit Fluidkanälen. Bevorzugt umfasst das Fluidsammelmittel einen offenporigen Polyurethanschwamm. Bevorzugt sind eine oder mehrere Drainageleitungen in dem Fluidsammelmittel befestigt. Bevorzugt sind die Drainageleitungen Schläuche. Bevorzugt besitzt die Drainageleitung am distalen Ende seitliche Drainageöffnungen. Bevorzugt ist die Drainageleitung am distalen Ende über die gesamte Länge, die sich in dem Fluidmittel befindet, mit seitlichen Drainageöffnungen versehen. Bevorzugt kann das Fluidsammelmittel In kleinere Drainagesystemeinheiten getrennt werden. Bevorzugt ist das Fluidsammelmittel zur Teilungsmöglichkeit in kleinere Drainageeinheiten mit Perforationsschlitzen versehen. Bevorzugt können die Drainageschläuche mit Verbindungselementen miteinander verbunden werden.

Zur Ableitung von Wundsekreten, Körperflüssigkeiten, Vereiterungen werden üblicherweise nach Operationen Drainageschläuche eingelegt. Diese sollen Sekrete über seitliche Öffnungen im Drainageverlauf im Sinne einer Schwerkraftdrainage und Überlaufdrainage ableiten. Drainagen können als Schlauchdrainage oder auch als flächige Drainagen konstruiert sein. Bei letzteren gelingt eine Drainage auch über eine Kapillarwirkung. Es werden auch Drainageschläuche eingelegt, die an einen Unterdruck angeschlossen werden. Die Wunddrainage entfalten ihre Wirkung meist aber nur unmittelbar nach einer Operation, da es unter anderem durch Fibrinausfällung, Koagulation von Blut und dem Anliegen von Gewebe zu einer schnellen Verstopfung der Drainageöffnungen kommt.

In der Behandlung von offenen sekundär heilenden Weichteilwunden hat sich in den letzten Jahren die Vakuumschwammtherapie etablieren können. Hierbei wird ein Polyurethanschwamm, der in eine Wunde eingelegt und mit einem Folienverband verschlossen wird, als Filter zur dauerhaften längerfristigen über Tagen durchzuführenden Wunddrainage genutzt. Die Wunden reinigen sich meist zügig und es gelingt insbesondere auch problematische Wunden zur Abheilung zu bringen. Auch zur Behandlung an inneren Wunden, die infolge von Anastomoseninsuffizienzen oder Perforationen am Darm entstehen, wurde die endoskopische Vakuumschwammtherapie mit Erfolg eingesetzt. Zur Behandlung von septischen Wunden in der Bauchhöhle bei z. B. bei einer Bauchfellentzündung werden ebenfalls Polyurethanschwämme zur Sekretableitung eingesetzt. Diese z. T. großflächigen Wundverbände werden mit einer Folie In Form eines Okklusionsverbandes am offenen Abdomen mit ein oder zwei Drainageansätzen unter einen Unterdruck gesetzt.

Die Erfindung birgt mehrere verbesserte und erweiterte Einsatzmöglichkeiten der Vakuumschwammtherapie. Bei der Erfindung handelt es sich um einen mit Drainageschläuchen versehenen offenporigen Schwamm, welcher sich in kleinere Untereinheiten teilen lässt. Die Drainageschläuche sind über ein Verbindungselement miteinander verbunden.

Dadurch, dass über die gesamte Länge eines Schwammelementes das perforierte Ende des Drainageschlauches im Schwamm befestigt ist, ist ein Vorteil der Erfindung, dass es nach der Anlage das Unterdrucks zum Zusammenziehen des Schwammes in der Breite aber nur unwesentlich zu einer Längenverkürzung kommt. Auf diese Weise bleibt die ursprüngliche Platzierung des Schwammes gesichert.

Bevorzugt ist der Kanal parallel zu einer Längsachse des Fluidsammelelementes angeordnet. Bevorzugt ist das Fluidsammelelement, insbesondere kreis-, zylindrisch ausgebildet und der Kanal Ist parallel zur Höhe des zylindrischen Fluidsammelelements angeordnet. Bevorzugt Ist die Längsachse des Fluidelementes diejenige, welche bei bestimmungsgemäßem Gebrauch des Fluidelementes im Wesentlichen parallel zu der Innenwand desjenigen Bereiches des Körpers orientiert ist, im welchem das Fluidsammelelement angeordnet ist. Ferner ist bevorzugt, dass der Kanal entlang einer Symmetrieachse des Fluidsammelelementes angeordnet ist. Bevorzugt ist das Fluidsammelelement röhrenförmig ausgebildet, wobei der Kanal der innere Hohlraum in dem röhrenförmigen Fluidsammelelement ist. Bevorzugt weist der Kanal eine erste Öffnung als Eingang für das Führungsselement und/oder das Versorgungselement und eine zweite Öffnung als Ausgang für das Führungsselement und/oder das Versorgungselement auf, Bevorzugt sind die erste und die zweite Öffnung auf sich im Wesentlichen gegenüberliegenden Oberflächen des Fluidsammelelementes angeordnet. Vorteilhaft wird erreicht, dass der Kanal zum Führen des Versorgungselements, insbesondere einer Sondeneinheit, durch das Fluidsammelelement genutzt wird, indem er eine Verbindung zwischen dem proximalen und distalen Ende des Fluidsammelelements bildet. Bei der symmetrischen Anordnung des Kanals im Fluidsammelelement werden die Vorteile erreicht, dass das Fluidsammelelement besonders gleichmäßig um einen beispielsweise in dem Kanal angeordneten Drainageschlauch kollabiert, falls dieser einen Unterdruck anlegt, dass Medikamente, die über ein in dem Kanal angeordnetes Versorgungselement dem Fluidsammelelement zugeführt werden, besonders gleichmäßig in dem Fluidsammelelement und auf dessen Oberfläche verteilt werden können, und dass die Führung entlang des Führungselementes besonders einfach ist, da das Fluidsammelelement mit identischem Radius um das Führungselement angeordnet ist. Eine außerhalb der Symmetrieachse aber zu dieser parallele Anordnung des Kanals bietet die Vorteile, dass ein bestimmten Bereich des Körpers, im welchem das Fluidsammelelement bei bestimmungsgemäßem Gebrauch angeordnet ist und welcher dem Kanal näher gelegen ist als der gegenüberliegende Bereich im Körper, den Einflüssen von Unterdruck, Medikamentzuführung etc. stärker ausgesetzt werden kann als der gegenüberliegende Bereich. Die parallele, insbesondere gerade, Anordnung des Kanals bietet den Vorteil, dass die Reibungskraft beim Führen des Fluidsammelelementes entlang des Führungselementes und beim Einführen des Versorgungselementes in das Fluidsammelelement vorteilhaft reduziert werden kann. Das Verschieben des Fluidsammelelementes gegenüber dem Versorgungselement wird so vorteilhaft vereinfacht.

Bevorzugt weist das Fluidsammelelement einen Längsschlitz entlang der Längsachse des Fluidsammelelementes auf, der sich zwischen der Oberfläche des Fluidsammelelementes und dem Kanal erstreckt. Bevorzugt erstreckt sich dar Längsschlitz zwischen den distalen und proximalen Enden und über die gesamte Länge des Fluidsammelelementes. Bevorzugt Ist der Längsschlitz in gleicher Weise wie der Längsschlitz des Overtubes ausgebildet. Bevorzugt erstreckt sich der Längsschlitz in radialer Richtung des Fluidsammelelements zwischen dem Kanal und der dem Kanal am nächsten gelegenen Oberfläche des Fluidsammelelementes. Ein längsgeschlitztes Fluidsammelelement bietet den Vorteil, dass es Jederzeit auf ein Endoskop, Führungselement, Versorgungselement etc. aufgesetzt und In den Körper eingeführt werden kann, ohne das Endoskop oder dergleichen entfernen zu müssen. Auch kann vorteilhaft erreicht werden, dass ein bereits in Benutzung befindliches Fluidsammelelement gegen ein frisches Fluidsammelelement ausgetauscht werden kann, ohne dass das Endoskop oder dergleichen während dieses Austauschs entfernt werden muss. Weitere Vorteile können in analoger Weise erreicht werden, wie sie für den längsgeschlitzten Overtube beschrieben sind. Bevorzugt wird der Längsschlitz im Fluidsammelmittel und/oder Overtube nach dem seitlichen Platzieren auf dem Führungs/Versorgungselement, insbesondere Endoskop oder Sonde, verschlossen, insbesondere mittels Naht, Klebung oder dergleichen. Verschiedene Verschlussmechanismen für das Fluidsammelmittel und/oder dem Overtube sind bevorzugt. Die Verschlussmechanismen sind ausgestaltet, um den Längschlitz vollständig oder nur teilweise (beispielsweise nur an der Spitze) zu verschließen, um ein Führen an dem Führungselement, beispielsweise Endoskop, zu vereinfachen. Ein vollständiger Verschluss ist beispielsweise durch eine Art Reissverschluss bevorzugt. Vorteilhaft kann so die Führung in körperöffnungsferne Bereiche und über Abknickungen erreicht werden.

Bevorzugt weist das Fluidsammelelement eine Röntgenmarkierung auf, die ausgestaltet ist, um bei einer Röntgenaufnahme detektiert zu werden. Bevorzugt ist die Röntgenmarkierung auf der Oberfläche des Fluidsammelelementes angeordnet. Bevorzugt ist die Röntgenmarkierung als Kreis um ein rotationssymmetrisch ausgestaltetes Fluidsammelelement angeordnet. Bevorzugt ist die Röntgenmarkierung im Inneren des Fluidsammelelements angeordnet. Die Anordnung einer Röntgenmarkierung an dem Fluidsammelelement weist den Vorteil auf, dass dessen Positionierung und auch die komplette Entfernung kontrolliert erfolgen und bei bestimmungsgemäßem Gebrauch der Vakuumschwammeinheit überwacht werden kann.

Bevorzugt Ist das Fluidkommunikationselement durch eine Fluidkommunikationselementöffnung in das Fluidsammelelement eingeführt und erstreckt sich entlang der vollständigen Länge des Fluidsammelelementes. Bevorzugt erstreckt sich das Fluidkommunikationselement entlang der vollständigen Längs- *und*/*oder* Symmetrieachse. Vorteilhaft wird erreicht, dass der Einfluss (Anlegen eines Unterdrucks/Überdrucks, Einführen eines Medikamentes etc.) des Fluidkommunikationselements über die gesamte Länge des Fluidsammelelementes im Wesentlichen identisch ist. Ferner wird erreicht, dass sich die Positionierung des Fluidsammelelementes, insbesondere des distalen Endes des Fluidsammelelements, im Wesentlichen nicht ändert, wenn das Fluidkommunikationselement einen Unterdruck, Überdruck, Medikament etc. an das Fluidsammelelement anlegt.

Erfindungsgemäß ist das Fluidkommunikationselement In dem Kanal angeordnet. Bevorzugt ist das Versorgungselement in dem Fluidkommunikationselement, insbesondere dem Drainageschlauch angeordnet. In einer bevorzugten Ausführungsform Ist eine Ernährungssonde in dem Drainageschlauch angeordnet und ausgestaltet, um vorgeschoben zu werden, wodurch auch noch nach der Platzierung des Fluidsammelelementes eine Sonde zur Ernährung durch den Kanal eingelegt, oder von vornherein so platziert werden kann, wodurch vorteilhaft Platz gespart wird. Je nach Art des zu versorgenden Bereiches des Körpers kann eine besonders kompakte Ausgestaltung der Vakuumschwammeinheit erreicht werden. Je nach anatomischer Ausgestaltung des zu versorgenden Bereiches und des Weges innerhalb des Körpers zu diesem Bereich kann die Anordnung von Fluidkommunikationselement zu Führungselement und/oder Versorgungselements vorteilhaft gewählt werden.

Erfindungsgemäß Ist das Fluidkommunikationselement ausgestaltet, um mit einer, elektrischen, Vakuumpumpe fluidleitend verbunden zu werden. Die Vakuumpumpe ist so ausgestaltet, um einen Unterdruck an das Fluidkommunikationselement anzulegen. Das Fluidkommunikationselement weist einen Adapter auf, um es direkt oder indirekt mit der Vakuumpumpe zu verbinden. Vorteilhaft wird erreicht, dass das Fluidsammelelement besonders effektiv und präzise mit einem Unterdruck beaufschlagt werden kann. Der Unterdruck ist kontinuierlich oder intermittierend. Da bei der endoskopischen Platzierung der Schwamm über den Sog fixiert wird, existiert immer ein bestimmter Unterdruck, sonst kann der Schwamm dislozieren. Es ist auch bevorzugt, dass der Unterdruck intermittierend angelegt wird, zwischen negativen Drücken schwankend (z. B. zwischen 80 und 125 mmHG). Das hat den Vorteil, dass der durch den Schwamm erzeugte Gewebedruck eine geringere mögliche Druckschädigung auf das Gewebe ausüben kann als bei erhaltener Fixierung durch den Negativdruck.

Bevorzugt ist das Fluidkommunikationselement fest mit dem Fluidsammelelement verbunden. Bevorzugt ist das Fluidkommunikationselement mit dem Fluidsammelelement verklebt, verschweißt, vernäht oder an diesem andersartig mechanisch fixiert. Vorteilhaft wird eine besonders zuverlässige Fluidleitung zwischen Fluidkommunikationselement und Fluidsammelelement erreicht. Ferner wird vorteilhaft erreicht, dass das Fluidsammelelement an dem Fluidkommunikationselement aus dem Körper gezogen werden kann.

Bevorzugt weist das Fluidkommunikationselement in demjenigen Bereich, welcher in dem Fluidsammelelement angeordnet ist, eine Vielzahl von Öffnungen auf. Bevorzugt sind die Öffnungen Perforationen. Bevorzugt sind die Öffnungen symmetrisch in der Oberfläche des Fluidkommunikationselementes angeordnet. Bevorzugt sind die Öffnungen nur auf einer Seite der Oberfläche des Fluidkommunikationselementes angeordnet, um ein asymmetrisches Kollabieren des Fluidsammelelements um das Fluidkommunikationselement zu erreichen, Mittels der Vielzahl von Öffnungen wird vorteilhaft eine im Wesentlichen gleichmäßige Verteilung des Einflusses des Fluidkommunikationselements auf den den Öffnungen gegenüberliegenden Bereich des Fluidsammelelements erreicht.

Bevorzugt weist die Vakuumschwammeinheit ein zusätzliches Fluidsammelelement auf, welches an dem Fluidkommunikationselement angeordnet ist. Bevorzugt weist das zusätzliche Fluidsammelelement die vorliegend beschriebenen Merkmale des Fluidsammelelementes auf. Bevorzugt sind das Fluidsammelelement und das zusätzliche Fluidsammelelement anliegend aneinander an dem Fluidkommunikationselement angeordnet. Vorteilhaft kann dadurch ein längerer Bereich des Körpers versorgt werden. Alternativ ist bevorzugt, dass das Fluidsammelelement und das zusätzliche Fluidsammelelement nicht anliegend aneinander (d.h. beabstanden voneinander) an dem Fluidkommunikationselement angeordnet sind. Vorteilhaft kann mit diesen Ausführungsformen erreicht werden, dass bei unterschiedlicher Ausgestaltung des zusätzlichen Fluidsammelelements und des Fluidelementes ein oder mehrere Bereiche im Körper unterschiedlich versorgt werden kann. Bevorzugt wird jedes Fluidsammelmittel einzeln unter einen Unterdrück gesetzt. Es kann auch eine lokale Spülbehandlung und/oder eine Saugbehandlung vorgenommen werden.

Bevorzugt Ist das Fluidsammelelement ausgestaltet, um ein Gas und/oder eine Flüssigkeit, welche(s) insbesondere Partikel aufweist(en), von einer äußeren Oberfläche des Fluidsammelelementes zu dem Fluidkommunikationselement (oder in umgekehrter Richtung (für Medikamente, Spülung etc,)) zu leiten. Bevorzugt ist das Fluidkommunikationselement ausgestaltet, um ein Gas und/oder eine Flüssigkeit, welche(s) insbesondere Partikel aufweist(en), von dem Fluidsammelelement abzuleiten, Bevorzugt ist das Fluidsammelelement ein offenporiger Polyurethanschwamm. Bevorzugt weist das Fluidsammelelement eine Porengröße von 200 µm bis 800 µm, Insbesondere bevorzugt von 400 µm bis 600 µm, auf, aber es sind auch andere Porengrößen möglich. Bevorzugt ist das Fluidkommunikationselement ein Drainageschlauch.

Bevorzugt weist das Vakuumschwammsystem ferner einen Overtube zum Umhüllen der Vakuumschwammeinheit, des Führungselements und/oder des Versorgungselements auf. Der Overtube weist einen kleineren Innendurchmesser als der Außendurchmesser des Fluidsammelelements auf. Der Overtube ist ausgestaltet, um das Fluidsammelelement zusammengedrückt zu führen. Der Overtube wird vorteilhaft verwendet, um das zusammengedrückte Fluidsammelelement entlang des Führungselementes, insbesondere des Endoskops, zu dem zu versorgenden Bereich innerhalb des Körpers zu führen.

Bevorzugt ist der Overtube entlang der Längsachse des Overtubes vollständig geschlitzt. Bevorzugt ist der Overtube zwischen proximalem und distalem Ende geschlitzt. Vorteilhaft wird erreicht, dass der Overtube jederzeit auf ein Führungselement und/oder Versorgungselement aufgesetzt oder von diesem entfernt werden kann.

Bevorzugt weist der Overtube ein erstes Ende, welches konisch ausgestaltet ist, und/oder ein zweites Ende auf, welches trichterförmig ausgestaltet ist. Bevorzugt ist das erste Ende das distale Ende und das zweite Ende das proximale Ende des Overtubes, Vorteilhaft wird erreicht, dass das distale Ende besonders einfach in den Körper eingebracht und bis zu dem zu versorgenden Bereich geführt werden kann. Durch das trichterförmig ausgestaltete Ende wird vorteilhaft erreicht, dass das Einführen von Führungselement, Versorgungselement, Vakuumschwammeinheit und dergleichen besonders einfach erfolgen kann.

Bevorzugt weist der Overtube wenigstens teilweise ein elastisch verformbares Material auf. Bevorzugt ist der Overtube aus Kunststoff gefertigt. Bevorzugt weist das Vakuumschwammsystem ferner eine Positionierungshülse und eine Führungshülse auf, wobei die Führungshülse gegenüber der Positionierungshülse verschiebbar ausgestaltet ist. Bevorzugt weisen die Positionierungshülse und/oder die Führungshülse wenigstens teilweise ein elastisch verformbares Material, insbesondere Kunststoff, auf. Vorteilhaft wird mit Overtube, Positionierungshülse und/oder Führungshülse ein besonders einfaches und sicheres Einführen und Positionieren der Vakuumschwammeinheit ermöglicht.

Erfindungsgemäß weist das Vakuumschwammsystem ferner eine elektrische, Vakuumpumpe auf, die mit dem Fluidkommunikationselement fluidleitend verbunden ist und ausgestaltet ist, um einen Unterdruck von bis zu 200 mmHg, bevorzugt 125 mmHg, an das Fluidsammelelement anzulegen. Bevorzugt Ist die Vakuumpumpe transportabel ausgestaltet, so dass der mit der erfindungsgemäßen Vakuumschwammeinheit versorgte Patient mobil ist. Das Versorgungselement ist im Verhältnis zu seinem Durchmesser lang und im Verhältnis zu seiner Länge dünn ausgestaltet. Erfindungsgemäß ist das Versorgungselement eine Ernährungseinheit, Bevorzugt dient das Versorgungselement auch als Führungselement zum Führen und Positionieren des Fluidsammelelements. Vorteilhaft wird erreicht, dass ein gesondertes Führungselement nicht erforderlich ist, um das Fluidsammelelement zu positionieren, so dass die Vakuumschwammeinheit besonders kompakt ausgebildet sein kann und ein einfaches Einführen in den Körper ermöglicht. Bevorzugt weist das Versorgungselement das Führungselement auf. Auch hierdurch kann eine besonders kompakte Ausgestaltung der Vakuumschwammeinheit erreicht werden.

Bevorzugte Ausführungsformen werdenim Folgenden anhand von Figuren erläutert, von denen
- Figuren 1 bis 7: ein Einführsystem zeigen,
- Figuren 8 bis 12: einen Overtube zeigen,
- Figur 13: eine Schwammdrainage zeigt,
- Figuren 14 bis 19: ein Drainagesystem zeigen.

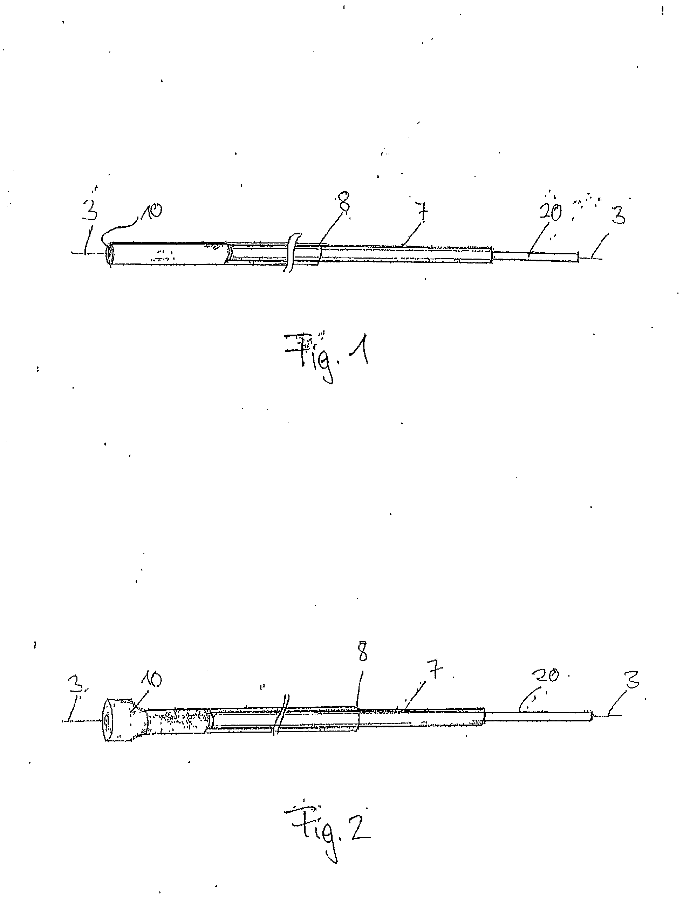

Eine Ausführungsform ist in Figuren 1 bis 7 gezeigt. Fig. 1 ist eine Darstellung, die die Anordnung des kompletten Einführsystems zeigt. Am distalen Ende der Führungshülse 8 ist der komprimierte Schwamm 10, der mit der Drainage 20 verbunden ist, aufgenommen, Über die Drainage 20 ist eine Positionierungshülse 7 ebenfalls in die Führungshülse eingeschoben, sie ist sowohl gegenüber der Führungshülse als auch der Drainage verschieblich.

In der Drainage liegt der Führungsdraht 3 ein. Dieser Führungsdraht wird zunächst endoskopisch über einen Defekt platziert, dann kann das ganze Einführungssystem über den Draht in die Position geschoben werden. Der Schwamm besitzt eine Röngtenstrahlen-dichte Markierung, so dass unter Röntgenkontrolle die Positionierung vorgenommen und kontrolliert werden kann.

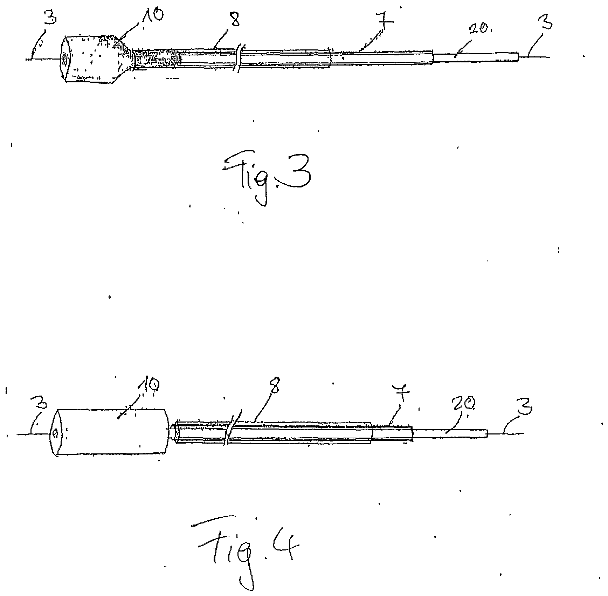

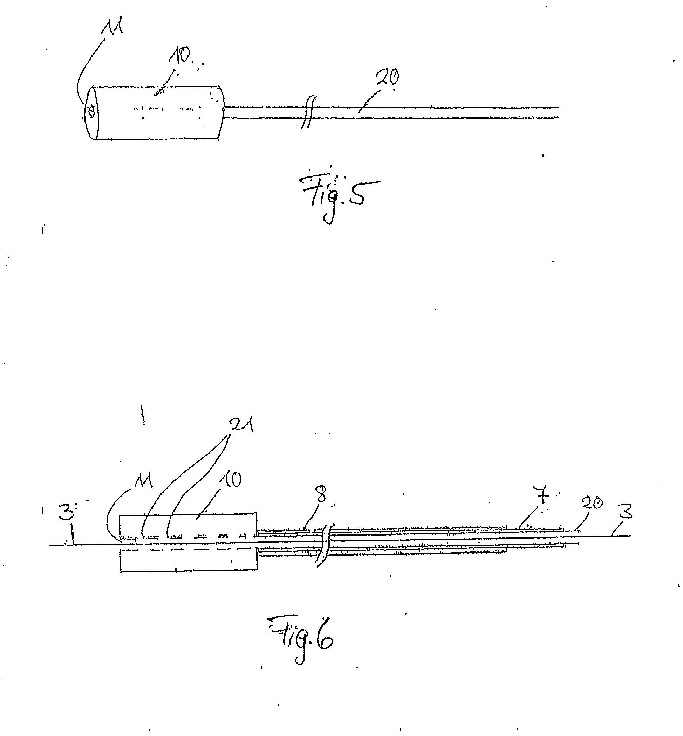

Fig. 2 zeigt das beginnende Freisetzen des Schwammes 10. Die Positionierungshülse 7 und die Führungshülse 8 werden gegeneinander verschoben, so dass der Schwamm aus dem Distalende der Führungshülse austritt. Fig. 3 zeigt ein fortschreitendes Freisetzen des Schwammes. Fig. 4 zeigt eine vollständige Schwammfreisetzung, Fig. 6 zeigt den freigesetzten Schwamm nach Entfernung der Führungshülse, Positionierungshülse und des Führungsdrahtes.

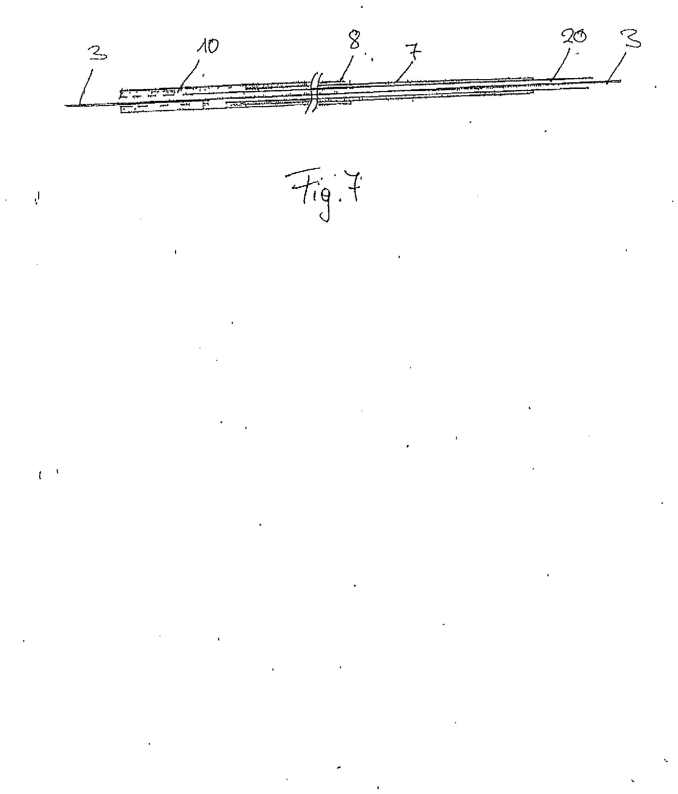

Fig. 6 zeigt einen Querschnitt durch das Einführungssystem mit freigesetztem Schwamm. 21 bezeichnet die Perforationsöffnungen der Drainage im Schwamm, Fig. 7 zeigt einen Querschnitt durch das Einführungssystem mit in dem Distalende der Führungshülse 8 komprimiertem Schwamm 10.

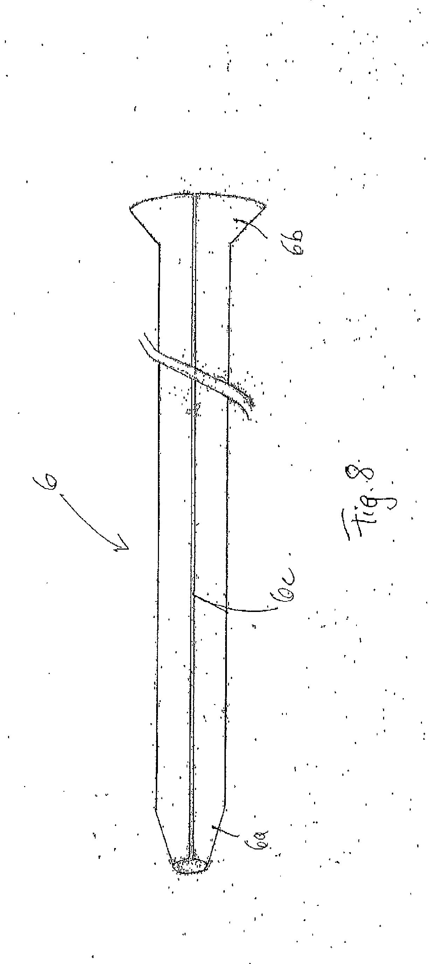

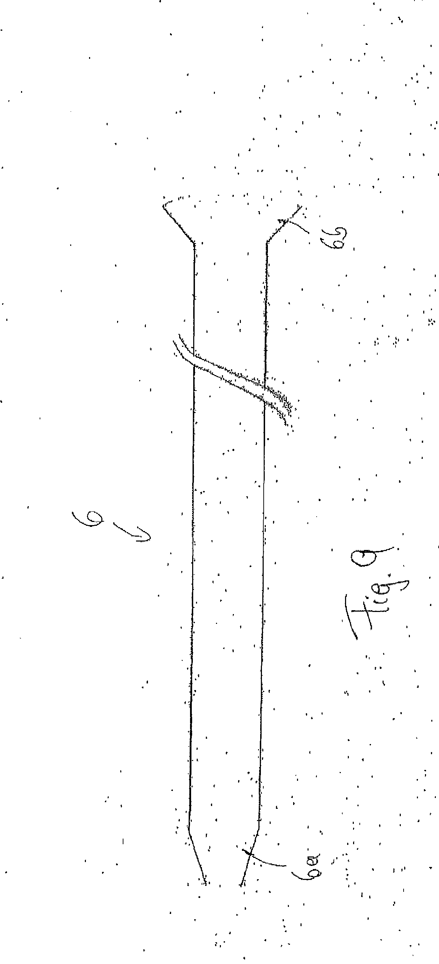

Eine weitere Ausführungsform ist in Figuren 8 bis 12 gezeigt. Fig. 8 ist eine Darstellung, die die Ausführung des Overtubes zeigt. Am Distalende 6a verjüngt sich der Overtube konisch, um Verletzungen beim Einführen zu vermeiden, Über die ganze Länge ist ein kompletter Schlitz 6c vorhanden. Am proximalen Ende 6b kann der Overtube trichterförmig konstruiert sein, um das Einführen der medizinischen Instrumente zu erleichtern. Fig. 9 ist eine Querschnittdarstellung mit konischem Distalende 6a und trichterförmigem proximalen Ende 6b.

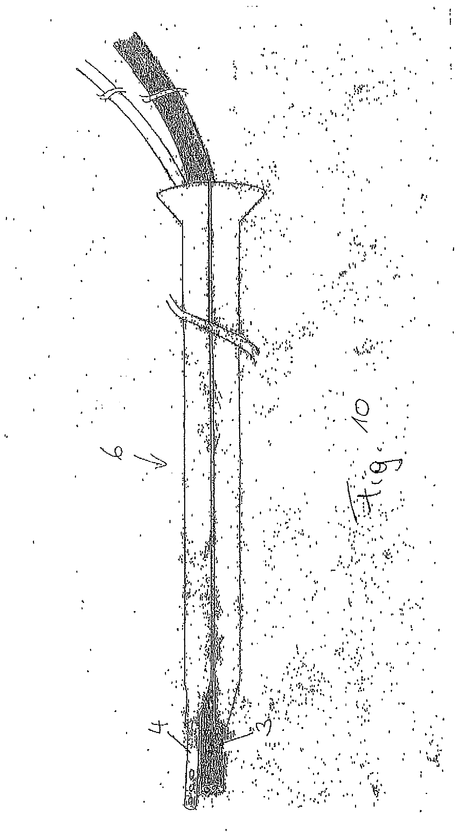

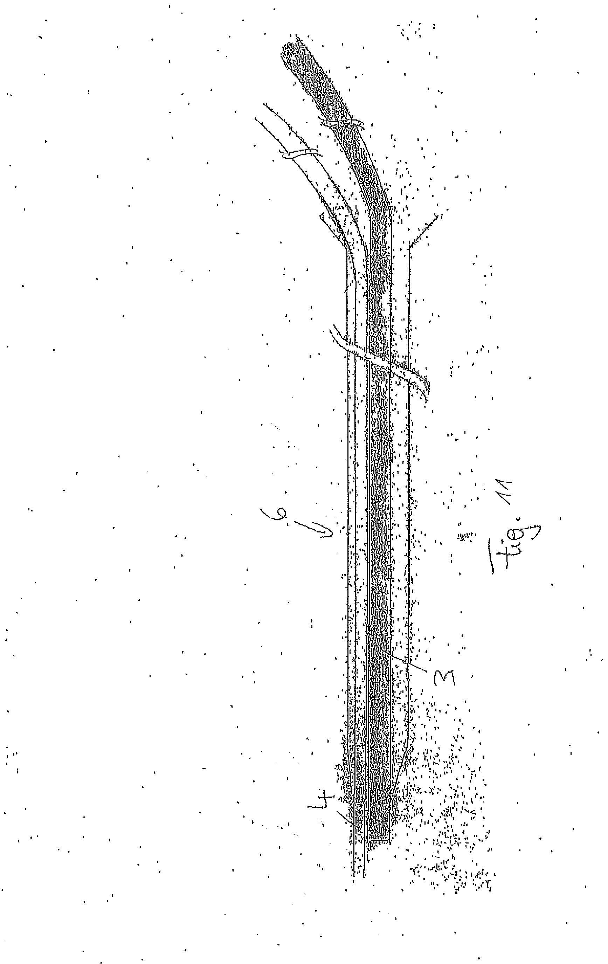

Fig. 10 zeigt wie eine Drainage 4 gemeinsam mit einem Endoskop 3 im Overtube eingebracht ist. Fig.11 ist eine Querschnittsdarstellung eines Overtubes mit einem eingebrachten Endoskop 3 und einer Drainage 4. Fig.

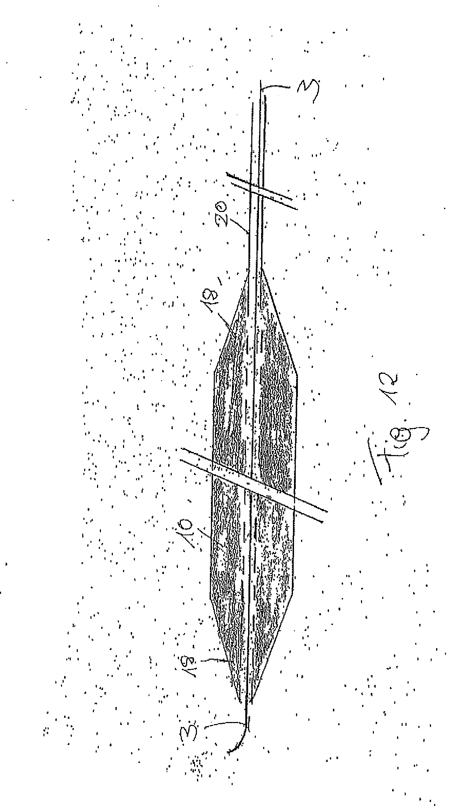

Fig. 12 ist eine Querschnittdarstellung eine Schwammdrainage mit konisch zulaufenden Enden 18 des Schwammkörpers 10, die an einer Drainage 20 mit seitlichen Perforationsöffnungen über die Länge des Schwammkörpers 10 befestigt wurde.

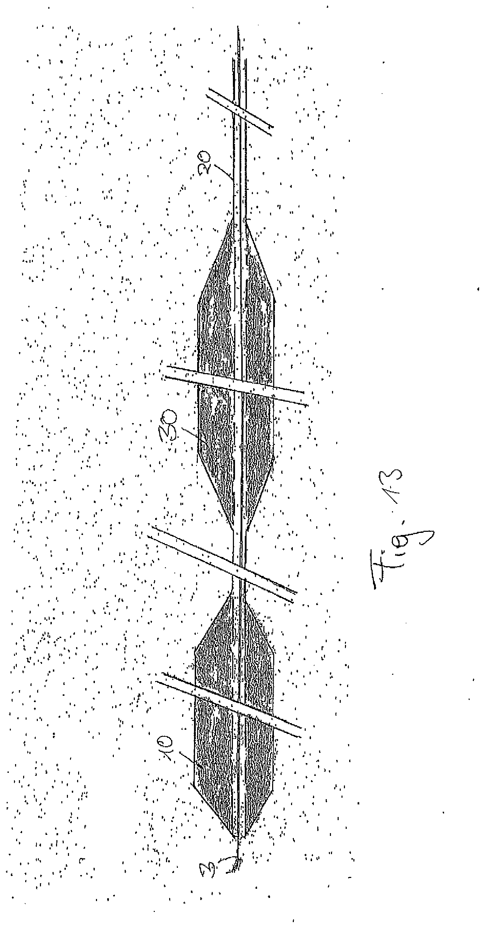

Führungsdraht 3 ist in der Drainage 20 angeordnet. Fig. 13 ist eine Querschnittdarstellung einer Schwammdrainage mit zwei konischen Schwammkörpern 10, 30, Führungsdraht 3 und Drainageschlauch 20.

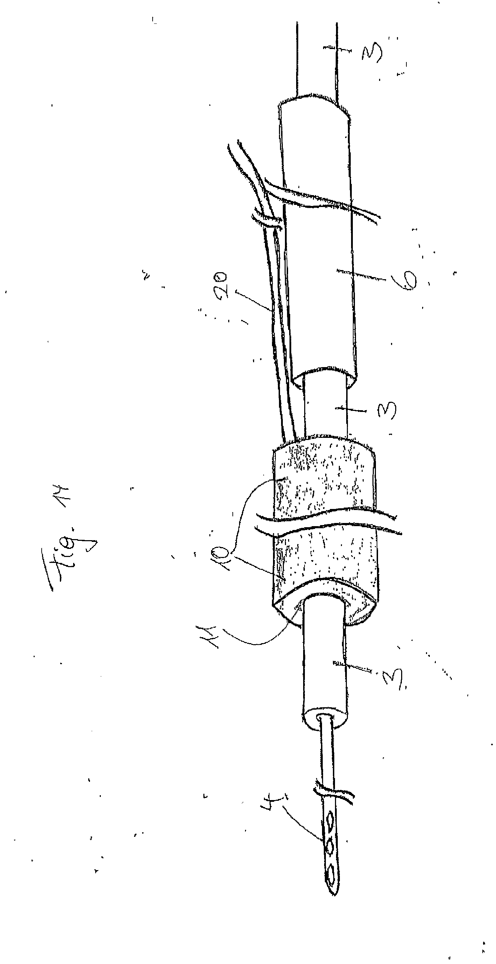

Eine Ausführungsform gemäß der Erfindung ist in Figuren 14 bis 19 gezeigt. Fig. 14 ist eine Darstellung, die die Anordnung des kompletten Drainagesystems (röhrenförmiger Schwamm 10 und Drainageschlauch 20) auf dem distalen Ende eines flexiblen durch einen Overtube 6 geführten Endoskops 3 zeigt. Durch den Arbeitskanal des Endoskops ist eine Ernährungssonde 4 bereits vorgeschoben worden.

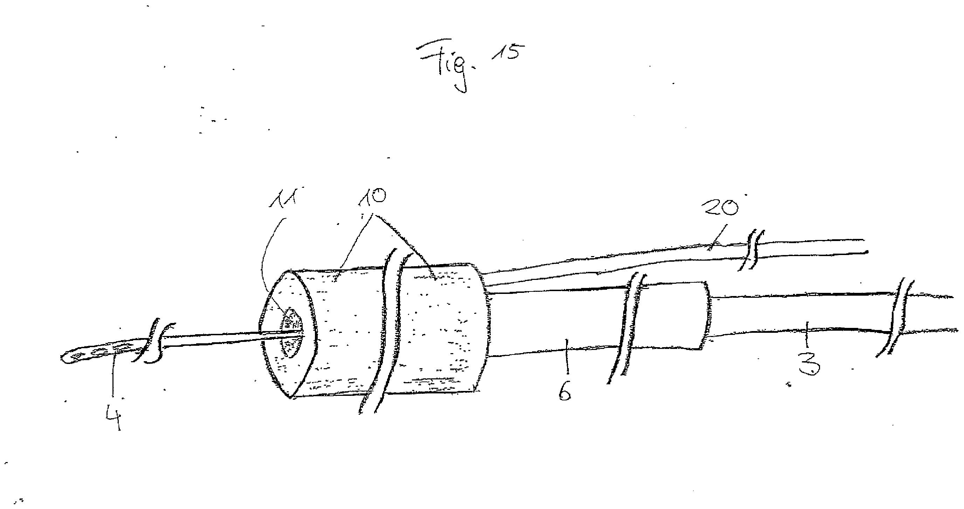

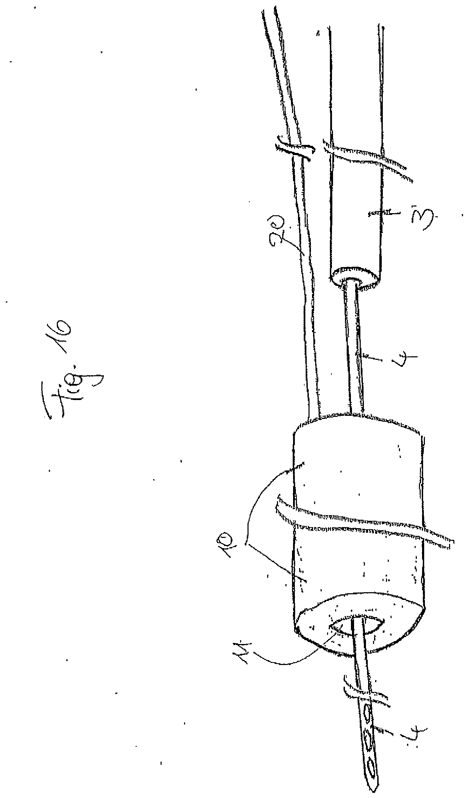

Fig. 15 zeigt die Handhabung der Freisetzung des Schwammes 10. Der Overtube 6 wird auf dem Endoskop 3 nach distal geführt. Der Schwamm rutscht über das distale Ende des Endoskops. Fig. 16 zeigt wie der Schwamm 10 endgültig freigesetzt ist. Zu diesem Zeitpunkt kann über den Drainageschlauch 20 ein Unterdruck angelegt werden. Durch den Unterdruck in einem Darmlumen oder in einer Höhle saugt sich der Schwamm fest, gleichzeitig wird die Ernährungssonde 4 im Schwamm fixiert.

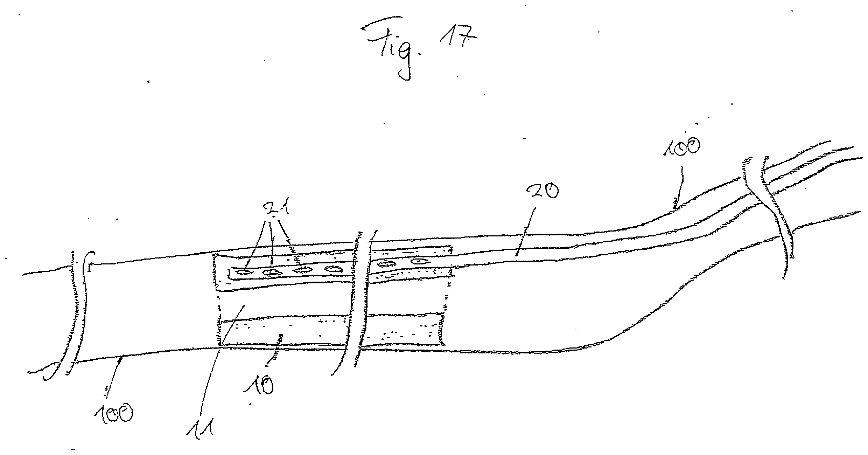

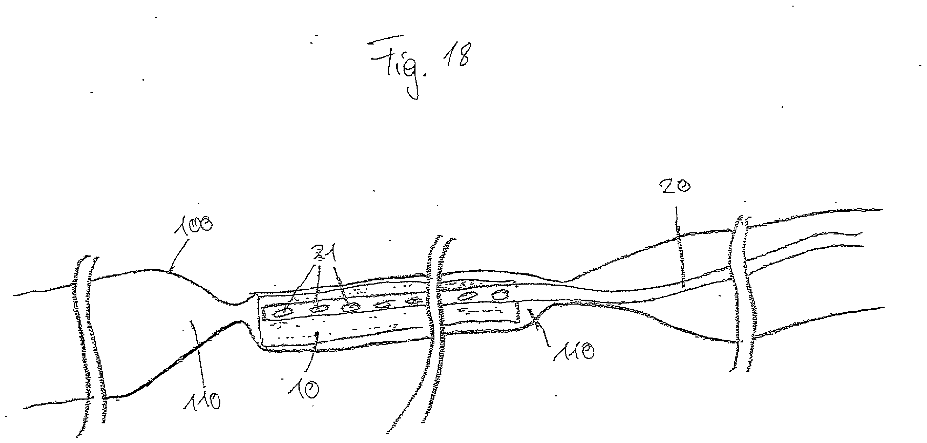

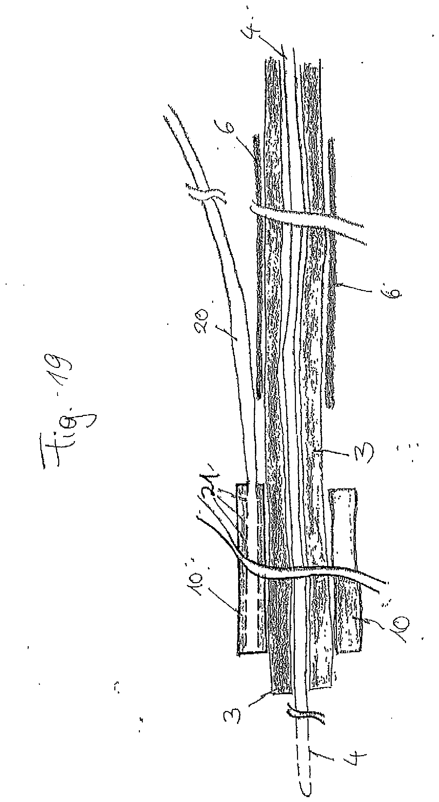

Fig. 17 zeigt eine Querschnittdarstellung eines in einem Darm 100 gelegten Schwamms 10 mit Drainageschlauch 20. Am distalen Ende des Drainageschlauches 20, im Schwamm 10 liegend, befinden sich seitliche Perforationsöffnungen 21. Fig. 18 zeigt einen in einem Darm 100 gelegten Schwamm 10, der unter einem Vakuum kollabiert ist. Hierdurch kollabiert auch das Darmlumen 110 am Schwamm 10 und führt zu einem 16 künstlichen Verschluss des Darmlumen 110. Am Distalende der Drainage 20 befinden sich Perforationsöffnungen 21. Fig. 19 ist eine Querschnittdarstellung des kompletten Drainagesystems (röhrenförmiger Schwamm 10, Drainageschlauch 20 mit Perforationsöffnungen 21 am Distalende) auf dem distalen Ende eines Endoskops 3 montiert. Das Endoskop 3 liegt in einem Overtube 6. Im Arbeitskanal des Endoskops 3 liegt eine Ernährungssonde 4.

## Patentansprüche

1. Vakuumschwammsystem, umfassend eine Vakuumschwammeinheit (2) zur Verwendung Im menschlichen oder tierischen Körper mit
einem Fluidsammelelement (10), insbesondere einer Schwammeinheit, und einem Fluidkommunikationselement (20), insbesondere einem Drainageschlauch,
welches(er) wenigstens teilweise in dem Fluidsammelelement (10) angeordnet und fluidleitend mit dem Fluidsammelelement (10) verbunden ist,
wobei das Fluidsammelelement (10) einen Kanal (11) zum Führen einer Ernährungssonde (4) durch das Fluidsammelelement (10) aufweist **gekennzeichnet durch** eine in dem Kanal in dem Fluidsammelelement zu führende Ernährungssonde, die im Verhältnis zu ihrem Durchmesser lang und im Verhältnis zu ihrer Länge dünn ausgestaltet ist, wobei das Fluidkommunikationselement (20) in dem Kanal (11) angeordnet ist und eine elektrische Vakuumpumpe (5), die mit dem Fluidkommunikationselement (20) fluidleitend verbunden ist und ausgestaltet ist, um einen Unterdruck von bis zu 200mmHg an das Fluidsammelelement (10) anzulegen.

2. Vakuumschwammsystem (2) nach Anspruch 1, wobei der Kanal (11) parallel zu einer Längsachse des Fluidsammelelementes (10) angeordnet ist.

3. Vakuumschwammsystem (2) nach wenigstens einem der Ansprüche 1 oder 2, wobei der Kanal (11) entlang einer Symmetrieachse des Fluidsammelelementes (10) angeordnet ist.

4. Vakuumschwammsystem (2) nach wenigstens einem der vorstehenden Ansprüche, wobei der Kanal (11) eine erste Öffnung (12) als Eingang für die Ernährungssonde (4) und eine zweite Öffnung (13) als Ausgang für die Ernährungssonde (4) aufweist.

5. Vakuumschwammsystem (2) nach Anspruch 4, wobei die erste und die zweite Öffnung (12, 13) auf sich im Wesentlichen gegenüberliegenden Oberflächen des Fluidsammelelementes (10) angeordnet sind.

6. Vakuumschwammsystem (2) nach wenigstens einem der vorstehenden Ansprüche, wobei das Fluidsammelelement (10) einen Längsschlitz (14) entlang der Längsachse des Fluidsammelelementes (10) aufweist, der sich zwischen der Oberfläche des Fluidsammelelementes (10) und dem Kanal (11) erstreckt.

7. Vakuumschwammsystem (2) nach wenigstens einem der vorstehenden Ansprüche, wobei das Fluidsammelelement (10) eine Röntgenmarkierung (15) aufweist, die ausgestaltet ist, um bei einer Röntgenaufnahme detektiert zu werden.

8. Vakuumschwammsystem (2) nach wenigstens einem der vorstehenden Ansprüche, wobei das Fluidkommunikationselement (20) durch eine Fluidkommunikationselementöffnung (18) in das Fluidsammelelement (10) eingeführt ist und sich entlang der vollständigen Länge des Fluidsammelelementes (10) erstreckt.

9. Vakuumschwammsystem (2) nach wenigstens einem der vorstehenden Ansprüche, wobei das Fluidkommunikationselement (20) fest mit dem Fluidsammelelement (10) verbunden ist.

10. Vakuumschwammsystem (2) nach wenigstens einem der vorstehenden Ansprüche, wobei das Fluidkommunikationselement (20) in demjenigen Bereich, welcher in dem Fluidsammelelement (10) angeordnet ist, eine Vielzahl von Öffnungen (21) aufweist.

11. Vakuumschwammsystem (2) nach wenigstens einem der vorstehenden Ansprüche, wobei die Vakuumschwammeinheit (2) ein zusätzliches Fluidsammelelement (30) aufweist, welches an dem Fluidkommunikationselement (20) angeordnet ist.

12. Vakuumschwammsystem (2) nach wenigstens einem der vorstehenden Ansprüche, wobei das Fluidsammelelement (10) ausgestaltet ist, um ein Gas und/oder eine Flüssigkeit, welche(s) insbesondere Partikel aufweist(en), von einer äußeren Oberfläche des Fluidsammelelementes (10) zu dem Fluidkommunikationselement (20) zu leiten, und/oder wobei das Fluidkommunikationselement (20) ausgestaltet ist, um ein Gas und/oder eine Flüssigkeit, welche(s) insbesondere Partikel aufweist(en), von dem Fluidsammelelement (10) abzuleiten oder zu dem Fluidsamenelement (10) zuzuleiten.

13. Vakuumschwammsystem (1) nach einem der vorhergehenden Ansprüche, wobei das Vakuumschwammsystem (1) ferner einen Overtube (6) zum Umhüllen der Vakuumschwammeinheit (2), und/oder der Ernährungssonde (4) aufweist.

14. Vakuumschwammsystem (1) nach Anspruch 13, wobei der Overtube (6) entlang der Längsachse des Overtubes (6) vollständig geschlitzt ist.

15. Vakuumschwammsystem (1) nach wenigstens einem der Ansprüche 13 oder 14, wobei der Overtube (6) ein erstes Ende (6a) aufweist, welches konisch ausgestaltet ist, und/oder ein zweites Ende (6b) aufweist, weiches trichterförmig ausgestaltet ist.

16. Vakuumschwammsystem (1) nach wenigstens einem der Ansprüche 13 bis 15 wobei der Overtube (6) wenigstens teilweise ein elastisch verformbares Material aufweist.

17. Vakuumschwammsystem (1) nach wenigstens einem der Ansprüche 13 ebis 16, wobei das Vakuumschwammsystem (1) ferner eine Positionierungshülse (7) und eine Führungshülse (8) aufweist, wobei die Führungshülse (8) gegenüber der Positionierungshülse (7) verschiebbar ausgestaltet ist.

## Claims

1. Vacuum sponge system, comprising a vacuum sponge unit (2) for use in the human or animal body with
a fluid collection element (10), in particular a sponge unit, and a fluid communication element (20), in particular a drainage tube,
which is arranged at least partially in the fluid collection element (10) and is connected in a fluid-conducting manner to the fluid collection element (10),
wherein the fluid collection element (10) has a channel (11) for guiding a feeding tube (4) through the fluid collection element (10), **characterized by** a feeding tube to be guided in the channel in the fluid collection element, which is configured to be long in relation to its diameter and thin in relation to its length, wherein the fluid communication element (20) is arranged in the channel (11), and an electric vacuum pump (5), which is connected in a fluid-conducting manner to the fluid communication element (20) and is configured to apply a negative pressure of up to 200 mmHg to the fluid collection element (10).

2. Vacuum sponge system (2) according to claim 1, wherein the channel (11) is arranged parallel to a longitudinal axis of the fluid collection element (10).

3. Vacuum sponge system (2) according to at least one of claims 1 or 2, wherein the channel (11) is arranged along an axis of symmetry of the fluid collection element (10).

4. Vacuum sponge system (2) according to at least one of the preceding claims, wherein the channel (11) has a first opening (12) as an inlet for the feeding tube (4) and a second opening (13) as an outlet for the feeding tube (4).

5. Vacuum sponge system (2) according to claim 4, wherein the first and the second opening (12, 13) are arranged on substantially opposite surfaces of the fluid collection element (10).

6. Vacuum sponge system (2) according to at least one of the preceding claims, wherein the fluid collection element (10) has a longitudinal slot (14) along the longitudinal axis of the fluid collection element (10), which extends between the surface of the fluid collection element (10) and the channel (11).

7. Vacuum sponge system (2) according to at least one of the preceding claims, wherein the fluid collection element (10) has an X-ray marker (15), which is configured to be detected during an X-ray exposure.

8. Vacuum sponge system (2) according to at least one of the preceding claims, wherein the fluid communication element (20) is inserted into the fluid collection element (10) through a fluid communication element opening (18) and extends along the entire length of the fluid collection element (10).

9. Vacuum sponge system (2) according to at least one of the preceding claims, wherein the fluid communication element (20) is fixedly connected to the fluid collection element (10).

10. Vacuum sponge system (2) according to at least one of the preceding claims, wherein the fluid communication element (20) has a multiplicity of openings (21) in that region which is arranged in the fluid collection element (10).

11. Vacuum sponge system (2) according to at least one of the preceding claims, wherein the vacuum sponge unit (2) has an additional fluid collection element (30), which is arranged on the fluid communication element (20).

12. Vacuum sponge system (2) according to at least one of the preceding claims, wherein the fluid collection element (10) is configured to conduct a gas and/or a liquid, which in particular comprises particles, from an outer surface of the fluid collection element (10) to the fluid communication element (20), and/or wherein the fluid communication element (20) is configured to discharge a gas and/or a liquid, which in particular comprises particles, from the fluid collection element (10) or to conduct it to the fluid collection element (10).

13. Vacuum sponge system (1) according to one of the preceding claims, wherein the vacuum sponge system (1) further has an overtube (6) for enclosing the vacuum sponge unit (2), and/or the feeding tube (4).

14. Vacuum sponge system (1) according to claim 13, wherein the overtube (6) is completely slotted along the longitudinal axis of the overtube (6).

15. Vacuum sponge system (1) according to at least one of claims 13 or 14, wherein the overtube (6) has a first end (6a), which is configured to be conical, and/or has a second end (6b), which is configured to be funnel-shaped.

16. Vacuum sponge system (1) according to at least one of claims 13 to 15, wherein the overtube (6) at least partially comprises an elastically deformable material.

17. Vacuum sponge system (1) according to at least one of claims 13 to 16, wherein the vacuum sponge system (1) further has a positioning sleeve (7) and a guide sleeve (8), wherein the guide sleeve (8) is configured to be displaceable with respect to the positioning sleeve (7).

## Revendications

1. Système d'éponge à vide, comprenant une unité d'éponge à vide (2) à l'usage dans le corps humain ou animal avec
un élément de collecte de fluide (10), en particulier une unité d'éponge, et un élément de communication de fluide (20), en particulier un tube de drainage,
qui est disposé au moins partiellement dans l'élément de collecte de fluide (10) et est relié de manière fluidique à l'élément de collecte de fluide (10),
dans lequel l'élément de collecte de fluide (10) présente un canal (11) pour guider une sonde d'alimentation (4) à travers l'élément de collecte de fluide (10)
**caractérisé par** une sonde d'alimentation à guider dans le canal dans l'élément de collecte de fluide, qui est conçue longue par rapport à son diamètre et mince par rapport à sa longueur, dans lequel l'élément de communication de fluide (20) est disposé dans le canal (11) et une pompe à vide électrique (5), qui est reliée de manière fluidique à l'élément de communication de fluide (20) et est conçue pour appliquer une dépression allant jusqu'à 200 mmHg à l'élément de collecte de fluide (10).

2. Système d'éponge à vide (2) selon la revendication 1, dans lequel le canal (11) est disposé parallèlement à un axe longitudinal de l'élément de collecte de fluide (10).

3. Système d'éponge à vide (2) selon au moins l'une quelconque des revendications 1 ou 2, dans lequel le canal (11) est disposé le long d'un axe de symétrie de l'élément de collecte de fluide (10).

4. Système d'éponge à vide (2) selon au moins l'une quelconque des revendications précédentes, dans lequel le canal (11) présente une première ouverture (12) en tant qu'entrée pour la sonde d'alimentation (4) et une deuxième ouverture (13) en tant que sortie pour la sonde d'alimentation (4).

5. Système d'éponge à vide (2) selon la revendication 4, dans lequel les première et deuxième ouvertures (12, 13) sont disposées sur des surfaces essentiellement opposées de l'élément de collecte de fluide (10).

6. Système d'éponge à vide (2) selon au moins l'une quelconque des revendications précédentes, dans lequel l'élément de collecte de fluide (10) présente une fente longitudinale (14) le long de l'axe longitudinal de l'élément de collecte de fluide (10), qui s'étend entre la surface de l'élément de collecte de fluide (10) et le canal (11).

7. Système d'éponge à vide (2) selon au moins l'une quelconque des revendications précédentes, dans lequel l'élément de collecte de fluide (10) présente un marqueur radiographique (15) qui est configuré pour être détecté lors d'une radiographie.

8. Système d'éponge à vide (2) selon au moins l'une quelconque des revendications précédentes, dans lequel l'élément de communication de fluide (20) est introduit à travers une ouverture d'élément de communication de fluide (18) dans l'élément de collecte de fluide (10) et s'étend le long de toute la longueur de l'élément de collecte de fluide (10).

9. Système d'éponge à vide (2) selon au moins l'une quelconque des revendications précédentes, dans lequel l'élément de communication de fluide (20) est relié de manière fixe à l'élément de collecte de fluide (10).

10. Système d'éponge à vide (2) selon au moins l'une quelconque des revendications précédentes, dans lequel l'élément de communication de fluide (20) présente une pluralité d'ouvertures (21) dans la région qui est disposée dans l'élément de collecte de fluide (10).

11. Système d'éponge à vide (2) selon au moins l'une quelconque des revendications précédentes, dans lequel l'unité d'éponge à vide (2) présente un élément de collecte de fluide supplémentaire (30) qui est disposé au niveau de l'élément de communication de fluide (20).

12. Système d'éponge à vide (2) selon au moins l'une quelconque des revendications précédentes, dans lequel l'élément de collecte de fluide (10) est configuré pour guider un gaz et/ou un liquide, qui présente(nt) en particulier des particules, depuis une surface extérieure de l'élément de collecte de fluide (10) jusqu'à l'élément de communication de fluide (20), et/ou dans lequel l'élément de communication de fluide (20) est configuré pour évacuer un gaz et/ou un liquide, qui présente(nt) en particulier des particules, depuis l'élément de collecte de fluide (10) ou pour l'acheminer jusqu'à l'élément de collecte de fluide (10).

13. Système d'éponge à vide (1) selon l'une quelconque des revendications précédentes, dans lequel le système d'éponge à vide (1) présente en outre un surtube (6) pour envelopper l'unité d'éponge à vide (2), et/ou la sonde d'alimentation (4).

14. Système d'éponge à vide (1) selon la revendication 13, dans lequel le surtube (6) est fendu complètement le long de l'axe longitudinal du surtube (6).

15. Système d'éponge à vide (1) selon au moins l'une quelconque des revendications 13 ou 14, dans lequel le surtube (6) présente une première extrémité (6a) qui est configurée sous forme conique, et/ou présente une deuxième extrémité (6b) qui est configurée sous forme d'entonnoir.

16. Système d'éponge à vide (1) selon au moins l'une quelconque des revendications 13 à 15, dans lequel le surtube (6) présente au moins partiellement un matériau élastiquement déformable.

17. Système d'éponge à vide (1) selon au moins l'une quelconque des revendications 13 à 16, dans lequel le système d'éponge à vide (1) présente en outre un manchon de positionnement (7) et une douille de guidage (8), dans lequel la douille de guidage (8) est configurée de manière déplaçable par rapport au manchon de positionnement (7).
